# EUROPEAN PATENT APPLICATION

(11) **EP 4 464 724 A1**
(43) Date of publication of application: **20.11.2024**
(21) Application number: 22919311.5
(22) Date of filing: 28.12.2022
(51) Int. Cl.: C07K 19/00, C07K 14/165, A61K 39/215, C12N 15/62, C12N 15/50, C12P 21/02, A61P 31/14

(54) **CHIMERIC PROTEIN PRODUCTION PROCESS, CHIMERIC PROTEIN, GENE, IMMUNOGENIC COMPOSITION AND USES**

(30) Priority: 14.01.2022 BR 102022000733
(71) Applicant: Universidade Federal de Minas Gerais, 31270-901 Belo Horizonte (BR); Fundação Oswaldo Cruz, 21045-900 Rio de Janeiro, RJ (BR); Fundação de Amparo à Pesquisa do Estado de Minas Gerais - Fapemig, 31035-536 Belo Horizonte - MG (BR)
(72) Inventor: TOSTES GAZZINELI, Ricardo, 31270-901 Belo Horizonte (BR); MARIA RIBEIRO TEIXEIRA, Santuza, 31270-901 Belo Horizonte (BR); SALLES MOURA FERNANDES, Ana Paula, 31270-901 Belo Horizonte (BR); GUIMARÃES DA FONSECA, Flávio, 31270-901 Belo Horizonte (BR); SALAZAR DE CASTRO, Natália, 31270-901 Belo Horizonte (BR); TEIXEIRA DE CASTRO, Júlia, 31270-901 Belo Horizonte (BR); SATCHIKO HOJO DE SOUZA, Natália, 30140-061 Belo Horizonte (BR); ORESTES DE AZEVEDO, Patrick, 31660-050 Belo Horizonte (BR); GOMES RAVELLI, Graziella, 34800-000 Caeté (BR); FONSECA BAGNO, Flávia, 31365190 Belo Horizonte (BR)
(74) Representative: Clarke, Modet y Cía., S.L.
(86) International application number: PCT/BR2022/050527
(87) International publication number: WO 2023/133617

(57) **Abstract**

The present technology discloses the process of producing a chimeric protein of SEQ ID No. 1, using the nucleotide sequence of SEQ ID No. 2. It also discloses the chimeric protein defined by SEQ ID No. 1, the gene of SEQ ID No. 2 used to its production, immunogenic compositions containing such protein and its use to prepare vaccines for the prophylaxis and prevention of infection with and moderate and severe forms of COVID-19. The present technology is included in the field of human health, specifically in the field of prevention measures against infection with SARS-CoV2. It addresses to the production of a vaccine composition comprising a chimeric protein that prevents high viral loads and moderate and severe clinical forms of the disease by stimulating the immune system.

## Description

The present invention is included in the field of human health, specifically in the field of prevention measures against SARS-CoV-2 infection. It addresses to a process of producing a chimeric protein, the chimeric protein, the gene used for its production and a vaccine composition comprising a chimeric protein that prevents high viral loads and moderate and severe clinical forms of the disease by stimulating the immune system. It also addresses to the uses of the chimeric protein and the immunogenic composition for the production of vaccines for the prophylaxis and prevention of infection with and moderate and severe forms of COVID-19.

Coronaviruses are species of viruses belonging to the *Coronavirinae* subfamily in the *Coronavidae* family. They are RNA viruses that infect humans and animals and cause respiratory, gastrointestinal or neurological disease. These viruses can emerge from animal reservoirs and cause significant epidemics in humans, such as Severe Acute Respiratory Syndrome (SARS-CoV), which occurred between 2002 and 2003, and Middle East Respiratory Syndrome (MERS-CoV), which emerged in 2012 in Saudi Arabia.

The pandemic caused by the new coronavirus 2019, COVID-19, has had a major impact on the world scenario, worsening morbidity and mortality rates and causing one of the biggest economic crises of the last hundred years. Given this scenario, global efforts are emerging to develop vaccines that are efficient in preventing infection by the pathogen.

Vaccines can be based on live virus, inactivated virus or viral components, such as purified surface antigens.

The vast majority of vaccines developed against the SARS-CoV-2 virus (which causes COVID-19) and all vaccines against SARS-CoV-2 that are already on the market use the Spike protein or the messenger RNA encoding this protein or even inactivated viruses, aiming at inducing neutralizing antibodies. Notably, the levels of antibodies against the Spike protein in the vaccines distributed in Brazil, after the first dose, are low. Furthermore, the most aggressive variants, with high transmissibility and possibly more pathogenic, have mutations in the Spike protein, which is the target antigen of the neutralizing antibodies, which makes currently available vaccines more vulnerable to these variations.

There is now significant evidence indicating an important role for T lymphocytes in protection against SARS-CoV-2 infection. To identify the targets recognized by these T lymphocytes, the present technology presents a virtual analysis that allowed the identification of the main peptides that interact with different HLAs (antigen-presenting molecules), identifying a high concentration of these peptides in the nucleocapsid (N) protein and in the RBD (Receptor Binding Domain) region of the Spike (S) protein.

To obtain an efficient and more effective combination of antigens against virus variants, the present technology uses an alternative protein, called nucleocapsid (N), which is the most abundant protein of SARS-CoV-2 and is highly immunogenic, as part of the chimeric protein developed in the present technology. This chimeric protein induces high levels of antibodies and production of IFNg by T lymphocytes, when inoculated into animals, and is also recognized by antibodies and T cells from convalescent individuals. The chimeric protein is composed of the N protein linked to the RBD segment of the Spike protein and, as it contains epitopes derived from the two viral proteins, its administration results in the production of antibodies against the RBD segment of the S protein and against the N protein.

The chimeric protein of the present technology is the association of the RBD portion of the Spike protein with the nucleocapsid (N) protein, and is therefore called SpiN. The RBD region binds directly to the Angiotensin Converting Enzyme (ACE-2) expressed on the surface of the host cell, thus being the main composition of most vaccines that are already being applied to the world population, in the form of RNA, non-replicating viral vectors or protein.

In a recent work, Matchett et al. (2021) describe a vaccine based on the N protein of SARS-CoV-2 expressed in a Human Adenovirus vector (Ad5), which confers protective immunity against infection in an animal model of COVID-19. (William E. Matchett et al. Nucleocapsid vaccine elicits spike-independent SARS-CoV-2 protective immunity. bioRxiv 2021.04.26.441518; doi: https://doi.org/10.1101/2021.04.26.44 1518).

In another study, Class et al. (2021) demonstrated that vaccination based on the Spike protein protects the lung in disease, but only protects animals from acute damage to the lung and brain in conjunction with a vaccine based on the Nucleocapsid protein (Jacob Class, Tanushree Dangi, Justin M. Richner, Pablo Penaloza-MacMaster. A SARS-CoV-2 nucleocapsid vaccine protects against distal viral dissemination. bioRxiv 2021.04.26.440920; doi: https://doi.org/10.1101/2021.04.26.440920) .

Patent document CN111607003, titled "SARS-CoV-2 N/S1 (RBD) recombinant protein and preparation method and application thereof", whose priority date is May 21, 2020, addresses to a recombinant protein comprising the gene fragment for expression of the nucleocapsid (N) protein of the SARS-CoV-2 virus and the gene fragment for expression of the RBD domain of the Spike (S1) surface protein of the SARS-CoV-2 virus, in addition to the expression vector, and the process of obtaining the protein for potential use as a vaccine for COVID-19. The protein obtained in document CN111607003 has a histidine tail in the N-terminal portion, normally introduced to facilitate the purification of recombinant proteins through a technique that requires the use of chelating agents. Chelating agents used in affinity chromatography to purify proteins containing amino acid tails such as histidine may present important disadvantages. The nickel used as a chelator to bind with histidine to purify the protein in CN111607003 is described in the literature as carcinogenic (Bal W, Kozlowski H, Kasprzak KS. Molecular models in nickel carcinogenesis. J Inorg Biochem 2000;79:213-8), and contamination with this metal may occur in purification processes of proteins produced by *E. coli* (Oswald T, Hornbostel G, Rinas U, Anspach FB. *Purification of ŽHis.6EcoRV recombinant restriction endonuclease EcoRV fused to a ŽHis.6 affinity domain by metal-chelate affinity chromatography. Biotechnol Appl Biochem 1997; 25ŽPt 2.:109-*15). Furthermore, it is demonstrated that histidine can act as a nickel-binding site on histone proteins, causing DNA damage (Zoroddu MA, Kowalik-Jankowska T, Kozlowski H, Molinari H, Salnikow K, Broday L, et al. *Interaction of NiŽII. and CuŽII. with a metal binding sequence of histone H4: AKRHRK, a model of the H4 tail. Bba Gen Subj* 2000;1475:163-8). It is worth highlighting that the nucleotide sequence disclosed in CN111607003 differs from the SpiN sequence. Based on these data, a sequence was produced for the SpiN protein that does not contain the additional histidine sequences and, for this reason, the purification process needed to be developed by using methodologies that involve intrinsic characteristics of the chimeric protein such as charge, molecular mass and solubility.

There are several strains of *E. coli* for expressing heterologous proteins. The BL21 (DE3) ΔSlyD pRARE *E. coli* strain (hereafter referred to as pRARE *E. coli*), encodes most of the tRNAs poorly expressed in *E. coli.* In turn, the SHuffle *E. coli* strain has an improved ability to provide better folding to proteins that contain multiple disulfide bonds in their structure, as is the case with the RBD portion of the SpiN protein.

The expression system of the present invention uses the pRARE or SHuffle *E. coli* strains and the pET-24a vector containing an optimized nucleotide sequence to obtain the chimeric protein of the present technology. Such a prokaryotic system has many advantages over eukaryotic systems, such as its simplicity and low cost, high level of expression and yield in a short space of time. The expression process of the present invention was optimized to obtain the protein with the highest yield and purity from the pRARE and SHuffle *E. coli* lysate. The protein does not have additional amino acids to those of the chimeric sequence itself, and is therefore purified through two chromatography steps. The strategy of uniting the sequences into a single molecule optimizes, simplifies, reduces time and cost of the process, when compared to the individual production of each sequence separately.

### BRIEF DESCRIPTION OF THE FIGURES

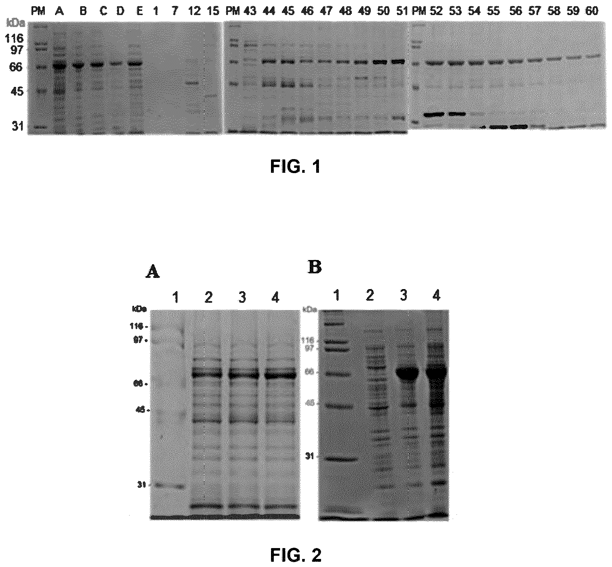

Figure 1: SDS-PAGE analysis of the result of SpiN purification by anionic chromatography (Channels A to E) followed by cationic chromatography (channels 1 to 60), from the soluble fraction of the *E. coli* protein extract (pRARE strain) (methodology 1). From left to right: (A) pre-anionic column sample containing SpiN; (B, C, D) sample not bound to the three-pass anionic column (unbound 1 to 3, respectively, pre-cationic column), (E) sample not bound to the cationic column, showing the large loss of SpiN protein (68 kDa), and (1 to 60) fractions eluted from the cationic column containing the SpiN protein. PM corresponds to the molecular weight standard.
Figure 2: Comparative SDS-PAGE of SpiN protein expression tests in (A) pRARE and (B) SHuffle *E*. *coli* strains. Channels 1 comprise the molecular weight standard; channels 2 comprise samples of non-induced strains; channels 3 comprise samples of strains induced for 3 h at 37 °C; channels 4 comprise samples of strains induced for 18 h at 20 °C.

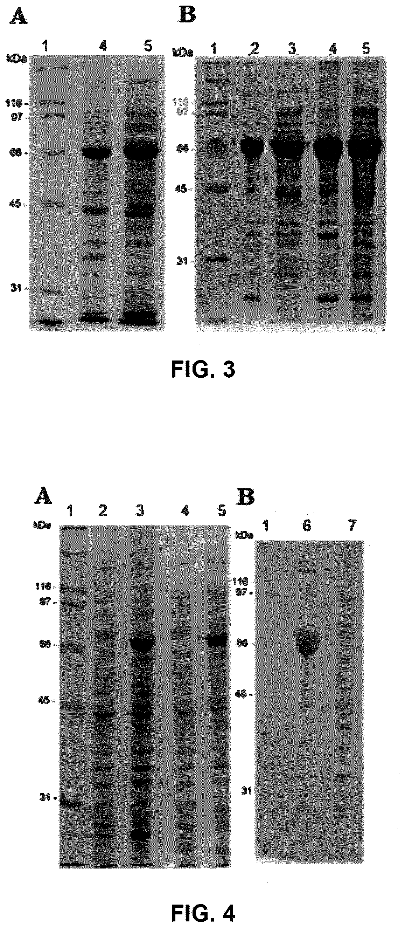

Figure 3: Comparative SDS-PAGE of SpiN protein solubility tests performed after expression in pRARE (A) and SHuffle (B) *E. coli* strains. Channels 1 comprise the molecular weight standard; channel 2 comprises a sample of the strain induced for 3 h at 37 °C, insoluble fraction; channel 3 comprises a sample of the strain induced for 3 h at 37 °C, soluble fraction; channels 4 comprise a sample of strains induced for 18 h at 20 °C, insoluble fraction; channels 5 comprise a sample of strains induced for 18 h at 20 °C, soluble fraction.
Figure 4: (A) Comparative SDS-PAGE of the SpiN protein expression induction test at 37 °C for 15 h, carried out on pRARE and SHuffle *E. coli* strains; (B) SDS-PAGE of the solubility test of SpiN expressed in the SHuffle strain. Channels 1 comprise the molecular weight standard; channel 2 comprises a sample of the uninduced pRARE *E. coli* strain; channel 3 comprises a sample of the pRARE *E. coli* strain induced for 15 h at 37 °C; channel 4 comprises a sample from the uninduced SHuffle strain; channel 5 comprises a sample of the SHuffle strain induced for 15 h at 37 °C; channel 6 comprises a sample of the SHuffle strain induced for 15 h at 37 °C, insoluble fraction; channel 7 comprises a sample of the SHuffle strain induced for 15 h at 37 °C, soluble fraction.

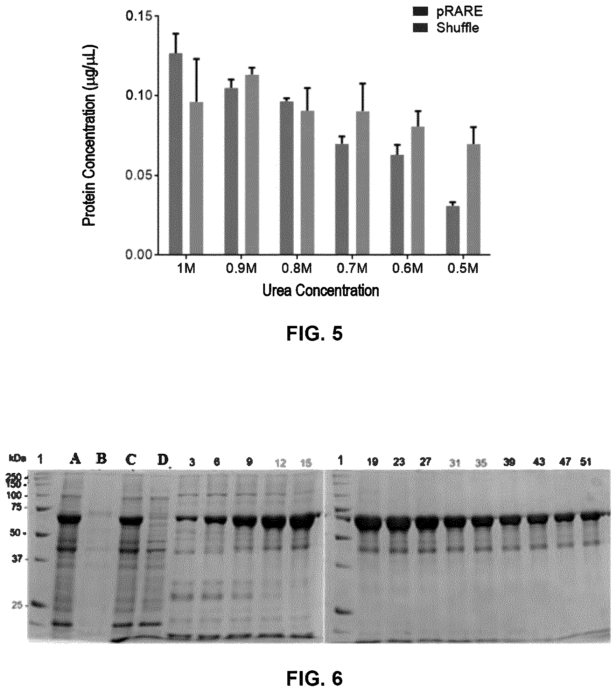

Figure 5: Evaluation of SpiN precipitation after reducing the urea concentration in pRARE and Shuffle *E. coli* strains.
Figure 6: SDS-PAGE analysis of SpiN purification from the insoluble fraction by anionic chromatography followed by cationic chromatography, according to methodology 2. Channels 1 correspond to the molecular weight standard; (A) SpiN protein sample obtained after solubilization (pre-anionic column); (B) sample eluted from the anionic column (anionic elution); (C) unbound from anionic column containing the SpiN protein (pre-cationic column); (D) unbound from cationic column; (3 to 51) fractions eluted from the cationic column.

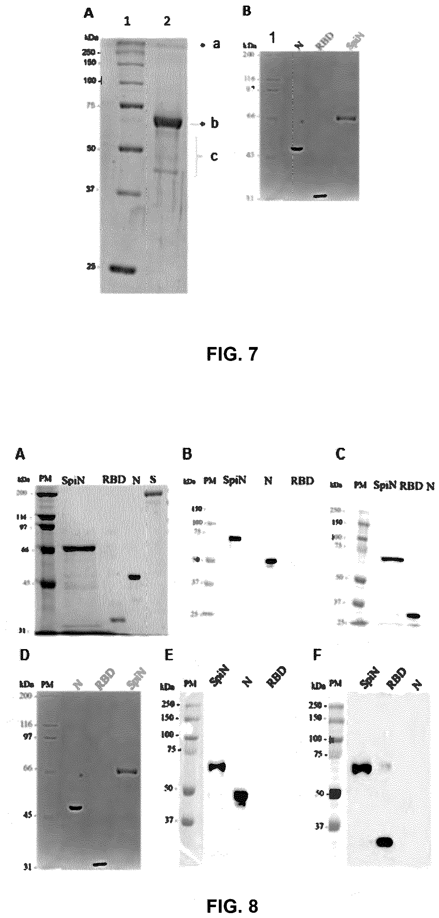

Figure 7: Analysis by SDS-PAGE of the final result of the purification of the SpiN protein following methodology 2. The (A) gel comprises, in channel 1 the molecular weight standard and, in channel 2, the SpiN protein after the two purification steps ("a" indicates SpiN aggregates, "b" indicates SpiN protein and "c" indicates SpiN cleavage products). The bands observed in the gel ("a", "b", "c") were sequenced by mass spectrometry, confirming the identity of SpiN ("b"), the presence of aggregates thereof ("a") and cleavage products ("c"). The (B) gel comprises the molecular weight standard (1), the purified N protein (N), the RBD portion of the spike protein (RBD), and the SpiN protein composed of N and RBD (SpiN).
Figure 8: SDS-PAGE showing the purified SpiN protein (A and D) and its recognition in Western blot performed with anti-N (B and E) and anti-RBD (C and F) antibodies. The purified N protein (N), the RBD portion of the Spike protein (RBD), and the Spike protein (S) were added as control. In the upper panels (A, B, C), the SpiN was purified following methodology 1. In the lower panels (D, E, F), the methodology 2 was used.

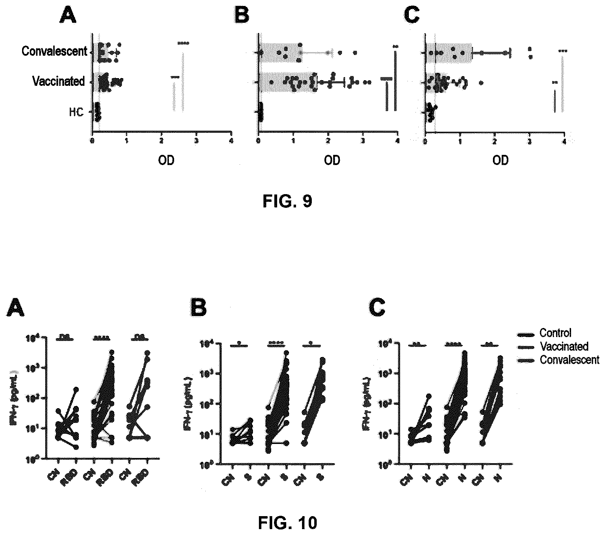

Figure 9: Levels of anti-RBD (A), anti-Spike (S) (B), and anti-nucleocapsid (N) (C) antibodies in the serum of unvaccinated and uninfected control individuals (HC, black circles), vaccinated with CoronaVac (green circles), and convalescents from infection with SARS-CoV-2 (red circles). Asterisks indicate that the difference is statistically significant (**, p < 0.01; ***, p < 0.001; OD = optical density).
Figure 10: Levels of IFN-g produced by peripheral blood mononuclear cells (PBMCs) stimulated by RBD (A), Spike (S) (B), and nucleocapsid (N) (C) proteins. PBMCs were purified in the blood of unvaccinated and uninfected control individuals (black lines), vaccinated with CoronaVac (green lines), and convalescents from SARS-CoV-2 infection (red lines). Asterisks indicate that the difference is statistically significant (**, p < 0.01; ***, p < 0.001). CN = negative control (PBMCs cultivated in the absence of any stimulus with SARS-CoV-2 antigens).

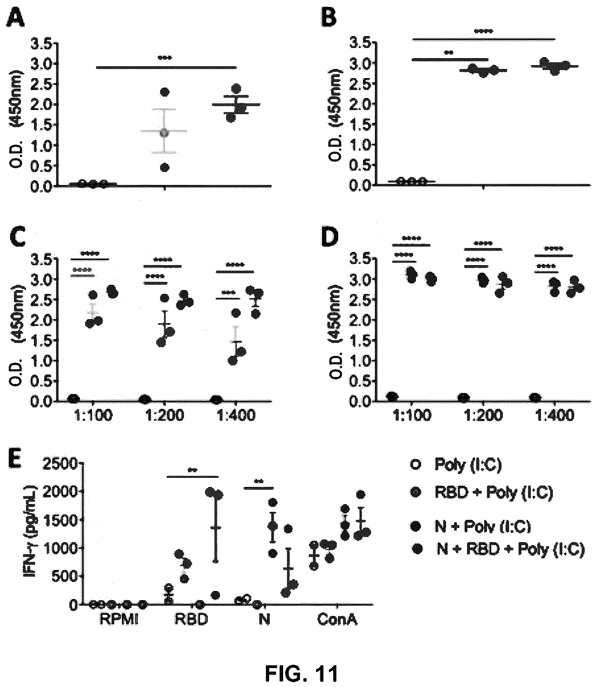

Figure 11: C57BL/6 mice immunized with N, RBD or N + RBD + Poly (I:C) were euthanized 21 days after the last vaccine dose, and the presence of anti-RBD and anti-N total IgG antibodies was evaluated in BALF (A and B, respectively) and in serum (C and D, respectively) through the ELISA assay. The cellular response was evaluated by detecting IFN-g in the culture supernatant of splenocytes stimulated with RBD or N. Concanavalin A (ConA) was used as a positive control (E).

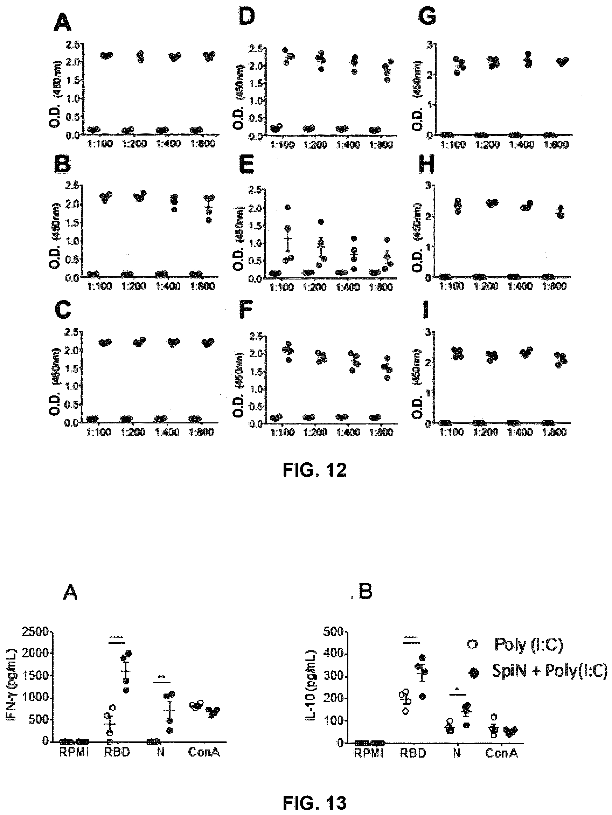

Figure 12: Humoral response of mice immunized with the SpiN + Poly (I:C) chimera. The total IgG (A, D and G), IgG1 (B, E and H) and IgG2c (C, F and I) anti-N (A, B, C), anti-RBD (D, E, F), and anti-SARS-CoV-2 (G, H and I) murine antibodies were measured in serum using the ELISA assay. The serum dilution is specified on the x-axis.
Figure 13: Splenocytes from mice immunized with SpiN + Poly (I:C) were stimulated with RBD or N for 48 h. The IFN-g (A) and IL-10 (B) cytokines were measured in the culture supernatant using the ELISA assay. ConA was used as a positive control.

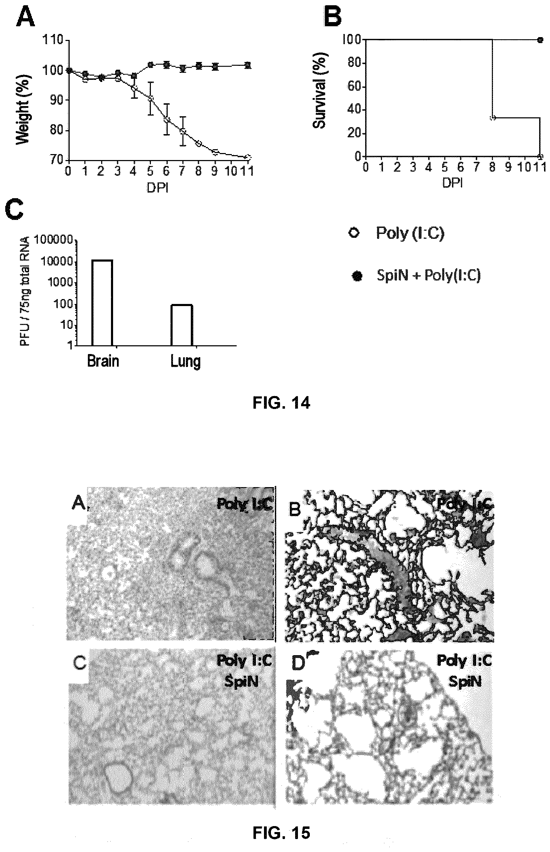

Figure 14: hACE2 Tg mice immunized with SpiN + Poly (I:C) Chimera were challenged with 5 x 10⁴ PFU of SARS-CoV-2. Weight (A) and mortality (B) were monitored for 11 days. The viral load in the brain and lung was evaluated by RT-PCR (C). DPI = days after infection.
Figure 15: Non-immunized hACE2 Tg mice that received only the immunological adjuvant (Poly (I:C)) (A, B) or immunized with SpiN + Poly (I:C) Chimera (C, D) were challenged with 5 x 10⁴ PFU of SARS-CoV-2 and the inflammatory process in the lung evaluated by histopathology. The lower and upper quadrants illustrate, respectively, the lung tissue from a vaccinated animal or an unvaccinated animal that received only Poly (I:C). Staining with hematoxylin and eosin (left quadrants) and Masson's Trichrome (right quadrants) illustrate the intensity of the inflammatory process and thrombus formation in the lungs of animals challenged with SARS-CoV-2, respectively.

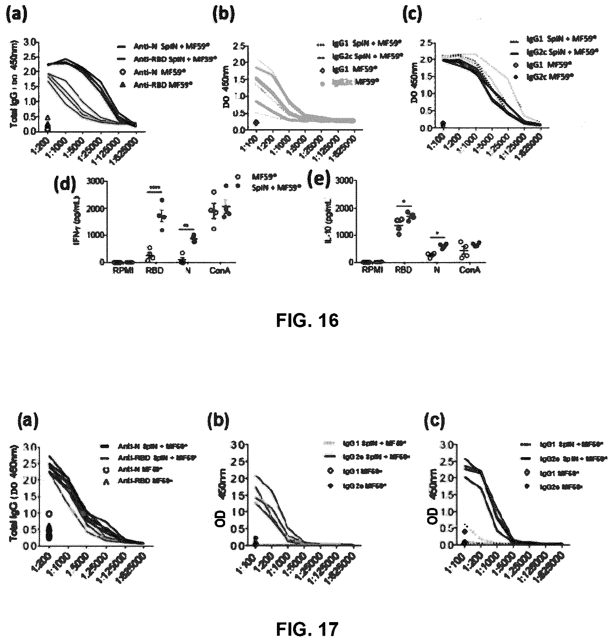

Figure 16: Levels of IgG (a), IgG1 and IgG2c anti-RBD (b) and anti-N (c) antibodies in mice immunized with SpiN associated with squalene adjuvant (commercial emulsion MF59). The serum dilution is specified on the x-axis. Levels of IFN-γ (d) or IL-10 (e) produced by splenocytes from vaccinated mice cultivated in the absence (RPMI) or presence of RBD, N or Concanavalin A (ConA, positive control).
Figure 17: Levels of total IgG (a), IgG1 and IgG2c anti-RBD (b) and anti-N (c) antibodies in hamsters immunized with SpiN associated with MF59. The serum dilution is specified on the x-axis.

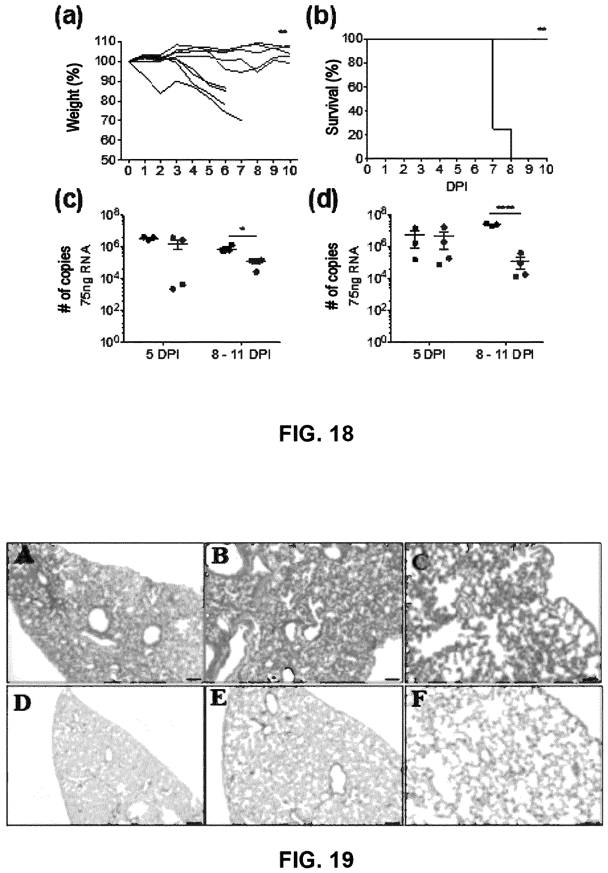

Figure 18: The protective effect of immunization of hACE2 mice with SpiN associated with MF59 adjuvant as indicated by weight loss (a), mortality (b) and viral load in the lung (c) and brain (d) 5 and 11 days after infection (DPI).
Figure 19: Histopathological sections of lungs from unvaccinated hACE-2 mice (A, B, C) and mice vaccinated with SpiN + MF59 (D, E, F) on the 5^{th} day after infection (DPI) with SARS-CoV-2. The tissue sections were stained with hematoxylin and eosin and analyzed for congestion, inflammatory infiltrates, foci of hemorrhage, intra-alveolar exudate, and alveolar collapse. Magnifications of 2.5 x (A and D); 4 x (B and E) and 20 x (C and F) .

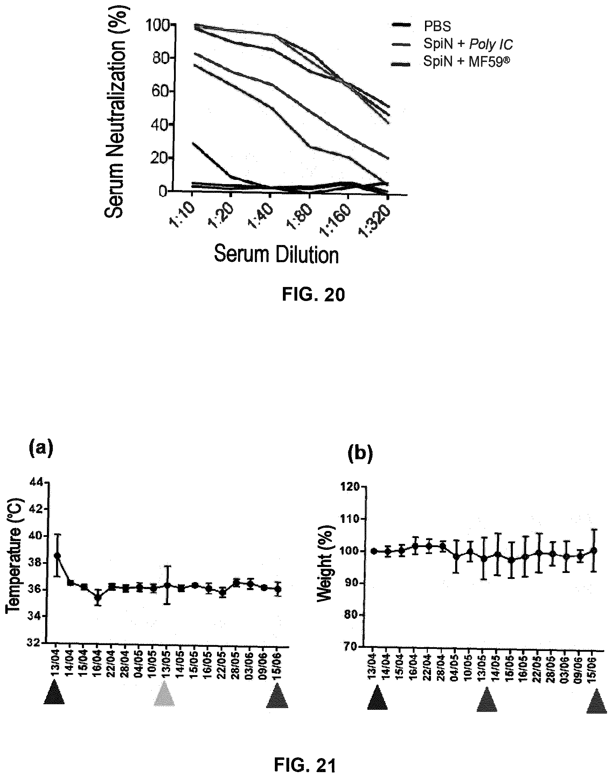

Figure 20: Serum neutralization assay with SARS-CoV-2 (Wuhan strain). Sera were collected from animals infected with SARS-CoV-2, unvaccinated control groups and vaccinated with SpiN + Poly (I:C) or SpiN + MF59, as indicated. The serum dilution indicates the neutralizing antibody titer in each sample.
Figure 21: Variation in temperature (a) and weight (b) of animals over time, after administration of SpiN + MF59. The red arrow indicates the 1^{st} dose, the blue arrow represents the 2^{nd} dose and the green arrow represents the end of the experiment.

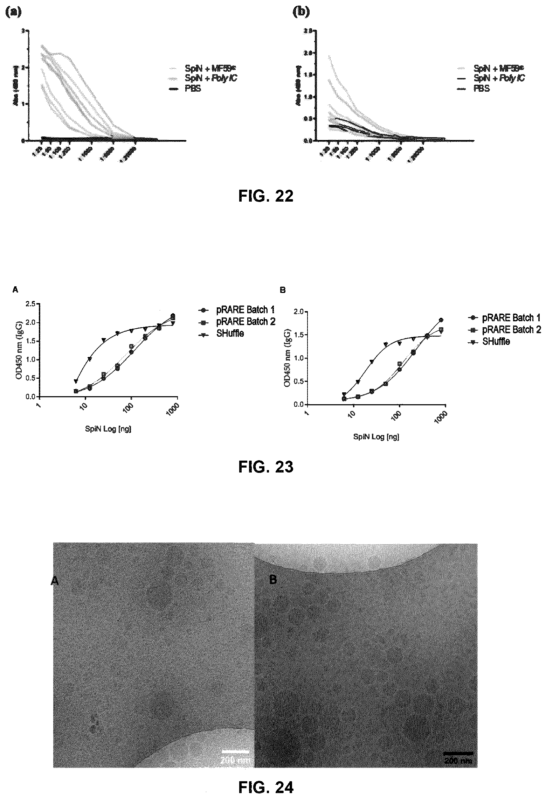

Figure 22: Anti-N (a) and Anti-RBD (b) total IgG evaluated 30 days after administration of SpiN associated with MF59. The serum dilution is specified on the x-axis.
Figure 23: SpiN protein titration for potency assay. Three batches of SpiN protein, produced in pRARE (Batch 1 and Batch 2) and SHuffle, were used as antigen in an indirect-type ELISA assay, being tested with specific polyclonal antibodies: (A) anti-N, and (B) anti-S/RBD, produced in rabbits. The serum dilution is specified on the x-axis.
Figure 24: Transmission electron cryomicroscopy of SpiN-AddaVax (A) and SpiN-squalene nanoemulsion (B).

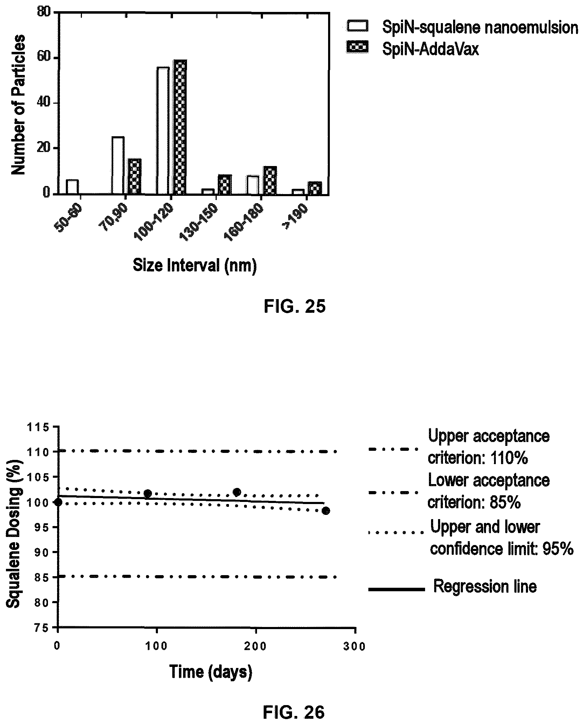

Figure 25: Size distribution analysis of SpiN-AddaVax and SpiN-squalene nanoemulsion particles obtained by microscopy. t-Student test, p < 0.05.
Figure 26: Evaluation of the squalene content of the immunogenic composition at 200 µg/mL stored at 5 °C ± 3 °C for 270 days.

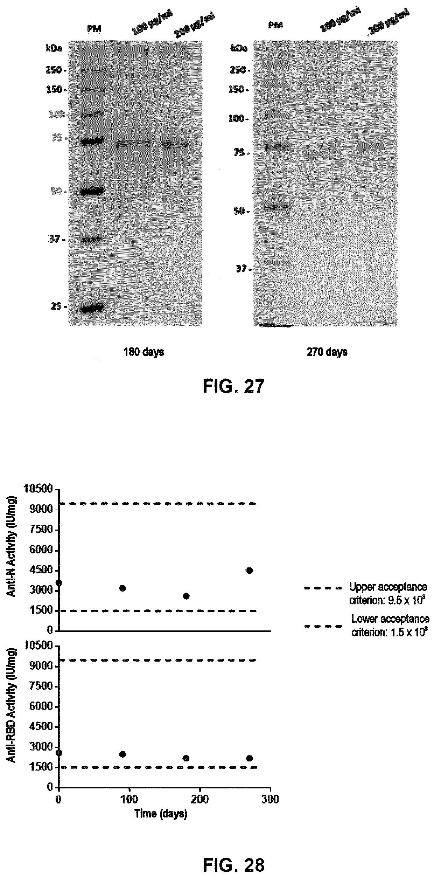

Figure 27: Analysis by DSS-EGPA, to evaluate the purity of the SpiN protein in the composition SpiN-squalene nanoemulsion 200 µg/mL, stored at 5 °C ± 3 °C, for 180 days and 270 days. PM indicates molecular weight standard in kDa.
Figure 28: Evaluation of the biological activity of SpiN in the immunogenic composition stored at 5 °C ± 3 °C for 270 days. Polyclonal antibodies produced in rabbits (anti-N and anti-RBD) were used to evaluate the biological activity of the two portions of the SpiN protein (N and RBD) contained in the SpiN-squalene nanoemulsion formulation by ELISA.

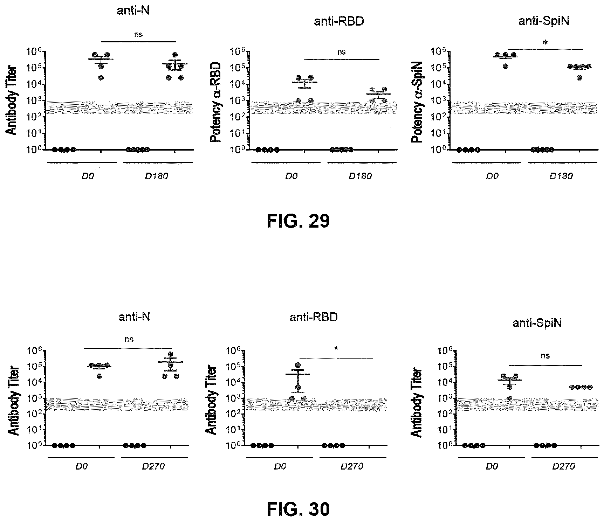

Figure 29: Evaluation of the potency of SpiN in the immunogenic composition stored at 5 °C ± 3 °C for 180 days. Humoral immune response in mice immunized with two doses of SpiN-squalene nanoemulsion on day 0 (D0) and 180 days after storage at 5 °C ± 3 °C (D180), against the control group (which received only the CTVad1 adjuvant). The results are expressed in anti-N, anti-RBD, anti-SpiN IgG antibody titers. On the y axis, the interval categorized as medium titers (in gray) was delimited, with low titers below the same and high titers above the same of total antibodies. The comparison between the groups was performed using the Mann-Whitney statistical test. * p < 0.05. ** p < 0.01. ns = not significant. The acceptance criterion for this test is equal to or greater than 10³.
Figure 30: Evaluation of the potency of SpiN in the immunogenic composition stored at 5 °C ± 3 °C for 270 days. Humoral immune response in mice immunized with one dose (20 µg) of SpiN-squalene nanoemulsion on day 0 (D0) and 270 days after storage at 5 °C ± 3 °C (D270) against the control group (which received only the adjuvant CTVad1, represented by black dots on the graph). The results are expressed in anti-N, anti-RBD, anti-SpiN IgG antibody titers. On the y axis, the interval categorized as medium titers (in gray) was delimited, with low titers below the same and high titers above the same of total antibodies. The acceptance criterion for this test is equal to or greater than 10² (after the first dose).

### DETAILED DESCRIPTION OF THE TECHNOLOGY

Unless defined differently, all technical and scientific terms used herein have the same meaning as understood by technician skilled on the subject to which the invention belongs. Conventional molecular biology techniques are well known to a technician skilled on the subject and can be found, for example, in Ausubel et al., eds. Current Protocols in Molecular Biology, John Wiley & Sons, Inc. N.Y. (1987-2008), including all supplements; Sambrook et al., Molecular Cloning: A Laboratory Manual, 2nd edition, Cold Spring Harbor, N.Y. (1989). The specification also provides definitions of terms to assist in the interpretation of what is described herein and the claims. Unless otherwise indicated, all numbers expressing quantities, percentages and proportions, and other numerical values used in the specification and claims, are to be understood as being modified, in all cases, by the term "about". Thus, unless otherwise indicated, the numerical parameters shown in the specification and in the claims are approximations that may vary depending on the properties to be obtained.

In a first aspect, the present invention refers to the process of producing a chimeric protein, defined by SEQ ID No. 1. To this end, the process involves the use of a nucleotide sequence defined by SEQ ID No. 2.

The chimeric protein production process comprises the following steps:
a. Cloning the DNA, defined by SEQ ID No. 2, encoding the protein defined by SEQ ID No. 1, into an expression vector;
b. Transforming *E. coli* bacteria (preferably the SHuffle *E. coli* strain) with the construction obtained in "a" and cultivating in an appropriate medium, such as LB medium with 25 to 100 ug/mL of kanamycin, at 36.5 to 37.5 °C, under stirring at 180 to 200 rpm, for 3 to 4 hours;
c. Inducing the expression of the protein defined by SEQ ID No. 1 by adding IPTG to the medium, preferably to a final concentration of 0.1 to 1 mM, when the optical density at 600 nm reaches the value of 0.3 to 0.6 and maintaining cultivation under stirring for a period of 15 h to 24 h, at 36.5 to 37.5 °C;
d. Lysing the cell precipitate from the cultivation obtained in "c", preferably using a lysis buffer, which comprises a buffering agent with a pH between 7 and 8, a cocktail of protease inhibitors and reducing agents, by passing the sample through homogenizing equipment by pressure maintaining 15,000 to 20,000 psi (103.42 to 137.90 MPa) for 5 to 20 min;
e. Submitting the lysate obtained in "d" to centrifugation at 35,000 g to 45,000 g for 20 to 40 min;
f. Performing 2 washing steps of the insoluble fraction obtained in "e" using preferably 80 to 100 mM Tris buffer, 0.5 to 1 M urea, 5 to 10 mM EDTA, 5 to 10% glycerol, 1 to 2% triton x-100, pH 7 to 8, through resuspension and centrifugation at 35,000 g to 45,000 g for 20 to 40 min;
g. Performing the precipitate resuspension step containing the washed insoluble fraction obtained in "f", preferably in 25 to 100 mM phosphate buffer, 5 to 10% glycerol, 6 to 8 M urea, 0.05 to 0.1% triton x-100, 1 to 10 mM DTT, 1 to 10 mM benzamidine, 1 to 5 mM PMSF, and protease inhibitor cocktail by stirring at 4 to 6 °C for approximately 18 to 24 h;
h. Submitting the resuspension obtained in "g" to centrifugation at 35,000 g to 45,000 g for 20 to 40 min;
i. Submitting the solubilized sample obtained in "h" to two steps of ion exchange chromatography, the first in an anionic column, and then the material not bound in the anionic column is applied to a cationic column, at pH 7 to 8, in the presence of 6 to 8 M urea;
j. Submitting the fractions with the highest concentration of the SpiN protein obtained in "i" to a desalting chromatography, to the remotion of the urea and preferential substitution for 25 to 100 mM phosphate buffer, 5 to 10% glycerol, 50 to 150 mM urea, 15 to 35 mM NaOH, 100 to 200 mM NaCl, pH 10.5 to 11.5.

In another embodiment, the expression cassette can be inserted into a variety of suitable vectors, such as, for example, plasmids, phages, viral or retroviral vectors. The technician skilled on the subject will know the most appropriate vector. Illustratively, if the microorganism to be modified is *Escherichia coli,* there can be used, for example, any commercial plasmid suitable for this host, such as, for example, the pET2a commercial plasmid.

The vector or cassette comprising the sequence of interest of the present invention is used to introduce these sequences into the host, by methods well known in the art, such as electroporation or thermal shock.

The polynucleotides of the invention can be inserted into a vector that contains transformation selection markers, such as antibiotic resistance genes.

In a second aspect, the present invention also refers to the chimeric protein defined by SEQ ID No. 1.

The chimeric protein obtained by the process described herein is characterized by comprising or consisting of the amino acid sequence defined by SEQ ID No. 1.

In a third aspect, the present invention also refers to the gene defined by SEQ ID No. 2.

The gene described herein is characterized by comprising or consisting of the amino acid sequence defined by SEQ ID No. 2.

In a fourth aspect, the invention relates to an immunogenic composition containing such protein and adjuvants and its use to prepare a vaccine for the prophylaxis and prevention of infection with and moderate and severe forms of COVID-19.

The adjuvants of the immunogenic composition are selected from the group comprising polyinosinic:polycytidylic acid (Poly (I:C)) and derivatives, water and oil emulsion or alum with unmethylated CpG oligonucleotide, water and oil emulsion or alum with lipid A, squalene and derivatives (such as squalene-based commercial nanoemulsions called MF59^{®} and AddaVax), synthetic TLR4 agonist, liposomes associated with unmethylated TLR CpG or TLR4 agonist, Montanide and Monophosphoryl Lipid A, or another adjuvant that induces an immune response of the Th1 type.

The immunogenic composition, in a preferred form, may comprise 1 µg to 1 mg of the protein defined by SEQ ID No. 1 and 1 µg to 10 mg of the polyinosinic:polycytidylic acid adjuvant, or 1 µg to 10 mg of CpG and 10% at 90% m/v alum, per dose.

In another preferred form, the composition may comprise 10 µg to 200 µg of the protein defined by SEQ ID No. 1 per dose, and 5 µg to 5 mg of the polyinosinic:polycytidylic acid adjuvant per dose, or 18 µg of CpG and 30% m/v of alum, per dose.

In another preferred form, the immunogenic composition may comprise 10 µg to 500 µg of the protein defined by SEQ ID No. 1, and 1 mg to 20 mg of the squalene adjuvant, per dose.

To exemplify one embodiment of the immunogenic composition containing the squalene adjuvant, the squalene-adjuvanted immunogenic composition may comprise 10 µg to 200 µg of the protein defined by SEQ ID No. 1 per dose, 0.004 µg to 0.1 µg of citric acid per dose, 0.04 mL to 1 mL of water for injections per dose, 0.07 µg to 1.4 µg of sodium citrate per dose, 0.3 µg to 4.5 µg of sodium chloride per dose, 1 mg to 25 mg of squalene per dose, 0.3 µg to 7 µg of dibasic sodium phosphate per dose, 0.03 µg to 0.7 µg of monobasic sodium phosphate per dose, 0.002 mL to 0.06 mL of glycerol per dose, 0.01 µg to 3 µg of polysorbate 80 per dose, 0.01 µg to 3 µg of sorbitan trioleate per dose, 0.1 to 3 µg of urea per dose, packaged in volumes between 1.0 and 5 ml.

In preferred forms, the adjuvanted immunogenic composition can be packaged in vials or ampoules under aseptic conditions.

In another example of a formulation for implementing the immunogenic composition containing the squalene adjuvant, the adjuvanted immunogenic composition may comprise 10 µg to 200 µg of the protein defined by SEQ ID No. 1 per dose, 0.3 µg to 4.5 µg of sodium chloride per dose, 0.3 µg to 7 µg of dibasic sodium phosphate per dose, 0.03 µg to 0.7 µg of monobasic sodium phosphate per dose, 0.002 mL to 0.06 mL of glycerol per dose, 0.1 to 3 µg of urea per dose and 0.02 mL to 0.5 mL of water for injections per dose filled in vials with volumes between 0.5 and 2.5 mL; the adjuvanted immunogenic composition may comprise 1 mg to 25 mg of squalene, 0.004 µg to 0.1 µg of citric acid per dose, 0.02 mL to 0.5 mL of water for injection per dose, 0.07 µg to 1.4 µg of sodium citrate per dose, 0.01 µg to 3 µg of polysorbate 80 per dose, 0.01 µg to 3 µg of sorbitan trioleate per dose, filled in vials with a volume between 0.5 and 2.5 mL and extemporaneously prepared by mixing the contents.

The chimeric protein of the present technology can be used to produce a vaccine for the prophylaxis and prevention of infection with and moderate and severe forms of COVID-19.

The immunogenic composition of the present technology can be used to produce a vaccine for the prophylaxis and prevention of infection with and moderate and severe forms of COVID-19.

The present invention is described by the nonlimiting examples below, which are merely illustrative. Various modifications and variations of the embodiments are evident to the technician skilled on the subject, without departing from the spirit and scope of the invention.

### EXAMPLE 1 - Construction pET-24a_N + RBD

The DNA encoding the N + RBD protein (SpiN), defined by SEQ ID No. 2, which was optimized for expression in *E. coli,* was cloned into the pET-14b bacterial expression vector (Genscript) and strategically subcloned into the pET24a vector to allow expression of the protein of interest without a histidine tail, and thus, without additional amino acids to those of the chimeric sequence itself. The pET24a expression vector confers resistance to kanamycin and contains the T7 phage (pT7) promoter.

### EXAMPLE 2 - Optimization of SpiN expression in E. coli BL21 (DE3) ΔSlyD pRARE bacteria and solubility test

Initially, *E. coli* BL21 (DE3) ΔSlyD pRARE (pRARE) bacteria were used, which were transformed with the pET-24a_SpiN construct and cultivated in LB medium with 50 µg/mL of kanamycin and 34 ug/mL of chloramphenicol a 37 °C under stirring at 200 rpm. The protein expression was induced by adding IPTG to the medium to a final concentration of 0.5 mM when the O.D. 0.6 at 600 nm was achieved. For analysis and comparison of the expression level of different colonies (clones), expression tests were carried out in two cultivation conditions: at 37 °C for 3 h and at 20 °C for 18 h. The clones that showed the best level of expression were cultivated and stored in the exponential phase of growth, with 25% glycerol in a volume of 1 mL (cryotubes) and stored at -80 °C.

To carry out a solubility test for the SpiN protein produced in pRARE *E. coli,* 1 mL of the contents of a stock cryotube from the cultivation was diluted in 4 mL of LB medium with 50 µg/mL of kanamycin and 34 ug/mL of chloramphenicol. The cultivation was maintained at 37 °C under stirring at 200 rpm for 18 h. Then, a 1:100 dilution was made in 250 mL of LB medium with the respective antibiotics in a 1 L Erlenmeyer flask. Growth continued at 37 °C under stirring until O.D. 0.6 at 600 nm was achieved. At this time, the protein expression was induced by the addition of IPTG to a final concentration of 0.5 mM. The cultivation temperature was reduced to 20 °C and continued for 18 h. After the induction time was complete, the cultivation was centrifuged at 8000 rpm for 10 minutes and the supernatant (medium) was discarded. The cell precipitate was then resuspended in 25 mL of lysis buffer (50 mM Phosphate, pH 7.7, 5 mM DTT, 5 mM benzamidine and 1 mM PMSF, the last three components were added at the time of use), and lysis of the cells made in the Emulsiflex-C3 homogenizer. Then, the sample was centrifuged at 40,000 g for 30 min. Samples of the insoluble fraction and the soluble fraction were analyzed. The protein was observed in similar proportions in both cell lysate fractions.

### EXAMPLE 3 - Optimization of the purification of the SpiN protein from the soluble fraction of pRARE E. coli (methodology 1)

The purification strategy developed here takes into account the fact that, due to its high isoelectric point (9.6), SpiN is protonated and positively charged in buffer with a pH below 9.6. Briefly, the methodology for purifying the SpiN protein from the soluble fraction consists of an anionic chromatography (to eliminate negatively charged contaminants) followed by a cationic chromatography (for binding and purification of SpiN).

To establish the SpiN purification protocol from pRARE *E. coli,* tests with different buffer solutions in the cell lysis and purification steps were carried out, mainly seeking to maintain the stability of the protein in the soluble fraction and optimize its binding to the cationic column in the second purification step. Additional tests, passing the sample through a single ionic (cationic and anionic) column and in different volumes, were carried out with the aim of optimizing the yield and purity of the process in each column. Finally, tests were carried out adding elution steps and gradients in cationic chromatography in order to optimize the elimination of contaminants. The purification steps were carried out following the manufacturer's manual for the columns used in the Akta prime (GE) system. The process established at the end of the optimization steps is described below.

In the first chromatography, three anionic columns - HiTrap Q XL (GE) - of 5 mL were used. Initially, the columns were equilibrated with 75 mL of 50 mM Phosphate buffer, pH 7.7, and then the sample was applied to the column at a flow rate of 1 mL per minute and the unbound fraction containing the SpiN protein (N + RBD) was collected. The run was completed at this step and the bound contaminants were eliminated in the column cleaning steps recommended by the manufacturer.

In the following chromatography, two 5 mL cationic columns - HiTrap SP HP (GE) - were connected. Initially, the columns were equilibrated with 50 mL of binding buffer (50 mM Phosphate Buffer, pH 7.7) at a flow rate of 5 mL per minute. Then, the sample containing the protein of interest with the highest degree of purity obtained from the previous step was applied at a flow rate of 1 mL per minute. After passing the sample, the columns were washed with 50 mL of binding buffer. The elution step was initially carried out in one step, passing through the column 50 mL of binding buffer with 20% elution buffer (50 mM Phosphate Buffer, pH 7.7, 1 M NaCl) and next there was made a linear gradient of 20% to 100% elution buffer in 50 mL. The sample eluted from the column was collected in 1.5 mL fractions and analyzed on a 10% SDS-PAGE gel. After purification analysis, the fractions containing the protein of interest were pooled.

By using the Bradford method, the yield of SpiN protein in the soluble protein extract, before the purification steps, was approximately 15 mg per 0.25 L of cultivation and, at the end of the process, approximately 4 mg per 0.25 L of cultivation. These yields were calculated for different batches produced after establishing the final protocol. However, even with all attempts to optimize the yield and purity of the protein, a very large loss of SpiN was observed in the unbound fraction of the cationic chromatography (second purification step), as well as the presence of protein bands smaller than SpiN, around 30 kDa, in elution fractions (Figure 1). Initially, it was believed that these bands were degradation products of the SpiN protein. However, the mass spectrometry sequencing carried out later showed that they were *E. coli* ribosomal proteins with an isoelectric point very close to that of SpiN and that, therefore, they had the same elution profile as the cationic column.

In addition to the presence of the contaminants described above, very rapid precipitation was visually noted in the sample during the purification steps. The precipitate was identified by SDS-PAGE as the SpiN protein. This fact led to the conclusion that the loss of the SpiN protein in the fraction that does not bind to the cationic column was occurring due to the precipitation thereof, resulting in the loss of the ability to interact with the column. From then on, a great search began for stabilizing reagents that could prevent this instability of SpiN in the soluble fraction of the bacterial lysate, which led to aggregation and precipitation. Although dozens of stabilizers have been tested (Table 1), promising results were obtained with only two of them: urea and sodium hydroxide. Urea, traditionally used to solubilize recombinant proteins expressed in *E. coli* inclusion bodies, showed good results at different concentrations (8 M to 2 M). Sodium hydroxide (in concentrations ranging from 10 mM to 30 mM) was able to break down SpiN protein aggregates and also keep it stable in its soluble form.

**Table 1 - Stabilizers tested for stabilization of the SpiN protein in the soluble fraction of the bacterial lysate.**

| **Tested Stabilizers** | **Concentration** |
|---|---|
| EDTA | 10 mM |
| Lactose | 2.5% |
| Glutathione | 20 mM |
| Glucose | 5% |
| Glycerol | 10%/15% |
| Sodium thioglycolate | 20 mM |
| Urea | 8 M/6 M/4 M/2 M |
| DTT | 5 mM/20 mM |
| Polysorbate 80 | 2% |
| Tween 80 | 2% |
| KCl | 200 mM |
| Glycine | 2% |
| Tween 20 | 2% |
| Mannitol | 5% |
| PEG 3350 | 6% |
| DMSO | 10% |
| MgCl₂ | 0.05% |
| Lysine | 0.5% |
| Trehalose | 15% |
| Sucrose | 15% |
| Sorbitol | 5% |
| Citrate | 100 mM |
| NaOH | 10 mM |

### EXAMPLE 4 - Comparative tests of expression and solubility of the SpiN protein in pRARE E. coli and SHuffle E. coli

In parallel to the tests with stabilizers, it was decided to evaluate expression and stability of the SpiN protein in another strain of *E. coli,* SHuffle. This strain was selected because it has an improved ability to provide better folding to proteins that contain multiple disulfide bonds in their structure, as is the case with the RBD portion of the SpiN protein. The SHuffle expression and solubility tests were carried out in exactly the same way as those carried out for the pRARE strain, with the only difference being the use of the antibiotic Kanamycin in the SHuffle strain's cultivation medium (without chloramphenicol).

Expression in different clones was evaluated for both strains. The comparative result of the expression test with induction of expression at 37 °C for 3 h and at 20 °C for 18 h shows a significant improvement in the expression level of SpiN in the SHuffle strain in both conditions (Figure 2, right panel). Another advantage of the SHuffle strain is the absence of SpiN expression escape before induction. In the pRARE strain, SpiN is expressed even before the addition of IPTG to the medium, which makes it difficult to visualize the induction of the expression and can harm cell multiplication and cultivation growth (Figure 2, left panel).

The solubility tests of the two strains showed the presence of the SpiN protein in both the soluble and insoluble fractions of the induction carried out at 20 °C for 18 h. For SHuffle, the induction was also carried out for 3 h at 37 °C and solubility was evaluated, again demonstrating the presence of SpiN in both fractions (Figure 3) .

With the aim of increasing cell yield at the end of cultivation and induction, and consequently the yield at the end of the SpiN purification process, a third induction condition was evaluated in both strains. It was decided to carry out the induction at 37 °C but increasing the time from 3 h to 15 h. After carrying out the test, the level of SpiN expression was evaluated. The results showed that increasing the cultivation time at 37 °C resulted in an approximately three-fold increase in cell yield when compared to the previous cultivation conditions. Furthermore, it was demonstrated that the level of SpiN expression was not impaired by the long induction time (which in some cases can result in degradation of the protein of interest) (Figure 4, left panel). Despite the promising results, the solubility test performed with the SHuffle strain indicated that the SpiN protein was produced only in the insoluble fraction (Figure 4, right panel).

### EXAMPLE 5 - Comparative stability tests of the SpiN protein in the insoluble fraction of pRARE E. coli and SHuffle E. coli after solubilization with urea

To define which strain to use in the SpiN protein production process, comparative stability tests with SpiN obtained from the insoluble fraction of both strains were carried out. After solubilization of SpiN in buffer with 8 M urea, the samples were subjected to gradual urea dilution steps (7 M - 6 M - 5 M - 4 M - 3 M - 2 M - 1 M - 0.9 M - 0.8 M - 0.7 M - 0.6 M - 0.5 M - 0.4 M - 0.3 M - 0.2 M - 0.1 M), reducing the urea concentration to acceptable levels for vaccine use. The dilutions came from SpiN protein samples at the same concentration. Until the dilution where the urea concentration reached 2 M, no protein precipitate formation was observed in any of the strains. In dilutions where the urea concentration was lower than 1 M, a precipitate was observed in the SpiN samples of both strains. In samples with a urea concentration lower than 1 M, the SpiN protein that remained in solution was quantified, wherein there was determined a higher concentration of SpiN in the soluble fraction of samples from the SHuffle strain and greater precipitation in the pRARE strain (Figure 5). In this way, the SHuffle strain was chosen as the expression cells for the production of the SpiN protein.

### EXAMPLE 6 - Optimization of the purification of the SpiN protein from the insoluble fraction of E. coli (methodology 2)

As in the stability tests previously carried out only urea and NaOH were capable of keeping SpiN soluble, it was defined that the purification steps would be carried out in buffer with 8 M urea and, next, the purified sample would be subjected to a buffer exchange for replacing the urea with NaOH, a reagent accepted in vaccine formulation buffers.

Thus, the final SpiN purification protocol was established from the cell pellet generated from a 1 L cultivation with induction of expression carried out at 37 °C for 15 h. The procedures of the cultivation step were carried out as previously described. As in this condition the protein was observed only in the insoluble fraction, in inclusion bodies, there were added washing steps of this fraction and solubilization in buffer with a high concentration of urea when compared to the previously described protocol for purifying SpiN from the soluble fraction. There follow details of the protocol, exemplified with SpiN produced in SHuffle *E. coli.*

The cell pellet generated from a 1 L cultivation was resuspended in 75 mL of 1X PBS, at the use moment, there were added 5 mM DTT, 5 mM benzamidine, 1 mM PMSF and an aliquot of protease inhibitor cocktail (amount indicated by the manufacturer). To lyse the cells, pressure homogenizing equipment was used and the sample was passed for 20 minutes under pressure varying between 15,000 and 20,000 psi (103.42 and 137.90 MPa). Then, the sample was centrifuged for 30 minutes at 40,000 g for clarification, the generated supernatant was discarded and the insoluble pellet containing the protein of interest was subjected to two washing steps to remove cell debris and insoluble *E*. *coli* proteins. The wash buffer containing 100 mM Tris, 1 M urea, 10% glycerol, 10 mM EDTA, 2% triton x-100, pH 7 was used to wash the pellet, which was then subjected to centrifugation for 30 minutes at 40,000 g. After centrifugation, the supernatant was discarded and another washing step was carried out in the same way. The washed pellet was then subjected to a solubilization step after resuspension in 75 ml of 50 mM phosphate buffer, 8 M urea, 10% glycerol and 0.05% triton X-100, pH 7.7 and incubated at 4 °C for 18 h under stirring. Next, the sample was centrifuged at 40,000 g for 30 minutes and the soluble fraction went to the purification steps.

As previously described, an anionic chromatography followed by a cationic chromatography was established to obtain SpiN protein with a high level of purity, as well as to remove bacterial endotoxins, as these can be eliminated due to the binding to anionic columns. In the first purification step, two 5 mL HiTrap Q XL (GE) anionic columns were used. Initially, the column was equilibrated with 25 mL of binding buffer (50 mM Phosphate Buffer, 10% glycerol, 8 M urea, pH 7.7) at a flow rate of 5 mL per minute. Next, the soluble sample containing the SpiN protein (obtained as described above) was applied to the column at a flow rate of 2 mL per minute. As the column has the same protein charge, the sample of interest is collected in the unbound fraction, allowing contaminants and endotoxins to bind and be eliminated at this step. In the second step, a 5 ml HiTrap SP HP (GE) cationic column was used. Initially, the column was equilibrated with 25 mL of binding buffer (50 mM Phosphate Buffer, 10% glycerol, 8 M urea, pH 7.7) at a flow rate of 5 mL per minute. Next, the sample collected from the unbound fraction from the previous chromatography was applied to the column at a flow rate of 2 mL per minute. After passing the sample, the column was washed with 25 mL of binding buffer. The elution step was performed with a gradient of 0 to 20% elution buffer (50 mM Phosphate Buffer, 10% glycerol, 8 M urea, 1 M NaCl, pH 7.7) flow rate of 2 mL per minute in 60 volumes column (300 mL) and at the end a step was applied with 100% elution buffer in 5 column volumes to elute the remaining bound material.

Figure 6 shows samples obtained from the two purification steps of methodology 2. The result of the cationic chromatography shows that there is no further loss of the SpiN protein in the sample that passes through the column without binding, as observed in methodology 1, indicating full utilization in the binding step. It was found that the loss at this step, observed in all cationic chromatographies described in methodology 1, was due to the sample aggregation. This result also indicates that a 5 mL HiTrap SP HP (GE) column is sufficient to be used with a 1 L cultivation, without saturation occurring.

To exchange the sample final buffer, with the aim of reducing the urea concentration, a HiTrap desalting column was used. The column was equilibrated with 1.5 column volumes of 50 mM phosphate buffer, 150 mM NaCl, 30 mM NaOH, 10% glycerol, and 100 mM urea, pH 11.2, and the sample was applied in a flow rate of 2 mL/min. The sample containing the SpiN protein was eluted from the column with the described equilibration buffer, defined as the protein final buffer. After this step, the pH was adjusted from 11.2 to 7.5 with 1 M phosphate buffer (5% of the sample volume). Stability tests showed that purified SpiN remains more stable at neutral pH. At pH 11.2, it was observed that SpiN is completely degraded when stored at 37 °C, which does not happen when adjusting the pH to 7.5.

After the buffer exchange and pH adjustment step, the purified SpiN was quantified and evaluated on SDS-PAGE. The bands observed in the gel were sequenced by mass spectrometry and the result showed that the majority band corresponds to the SpiN protein, the lower bands to cleavage products of the same and the higher ones to aggregates (Figure 7). The quantitative analysis of host cell proteins resulted in non-detectable values, showing a very high degree of purity of SpiN. In the quantitative analyses of endotoxins, the result observed was very low values, approximately 0.31 EU/mL, showing the effectiveness of anionic chromatography in removing this bacterial contaminant. The yield observed at the end of the purification steps is approximately 20 mg of SpiN protein per liter of cultivation with an excellent degree of purity.

### EXAMPLE 7 - In vitro SpiN protein recognition test (N + RBD)

SDS-PAGE of the purified SpiN protein (Figure 8, A and D). In the upper panels, SpiN was purified following the methodology 1. In the lower panels, the methodology 2 was used. Western blot with anti-N polyclonal serum (B and E) and anti-RBD monoclonal antibody (C and F) were performed to analysis of purified proteins. The anti-N polyclonal serum recognizes the SpiN chimera and the N protein, but not the RBD protein. In turn, the anti-RBD monoclonal antibody recognizes the SpiN chimera, the RBD and S proteins, but not the N protein.

### EXAMPLE 8 - In vitro assays: antibody response of uninfected, vaccinated and convalescent individuals against RBD and N proteins

To ensure that N or RBD proteins trigger humoral and cellular responses, samples from convalescent COVID-19 patients or individuals who were immunized with the inactivated virus vaccine (CoronaVac^{®}) were used.

Figure 9 shows the antibody response of uninfected individuals (HC), vaccinated with CoronaVac and convalescent from COVID-19 against the RBD (Figure 9A) and N (Figure 9C) proteins, separately. Full-length S protein was used as a positive control (Figure 9B). Total antigen-specific IgG antibodies were measured in the individuals' serum by using the ELISA method. A slight but significant increase in antibodies against the RBD proteins and a more significant increase in antibodies against the N protein were observed in vaccinated or convalescent patients. The level of antibodies against S protein was very high, both in vaccinated individuals and in convalescent individuals.

The production of IFN-g by PBMCs from these same patients was quite high for the three antigens (Figure 10), indicating that the selection of these proteins was consistent with our virtual analysis and relevant to the immune response of anti-SARS-CoV-2 patients. Overall, both convalescent and vaccinated individuals, but not seronegative healthy controls, showed a robust IFN-γ production response to the recombinant N and RBD proteins that make up our chimeric protein.

### EXAMPLE 9 - Humoral and cellular response of mice immunized with the N, RBD, N + RBD and SpiN proteins adjuvanted with Poly (I:C)

The vaccine potential of the SpiN protein was evaluated in a murine model. Initially, C57BL/6 mice were immunized with RBD or N alone, or with a combination of N + RBD, adjuvanted with polyinosinic:polycytidylic acid (Poly (I:C)). Two doses were administered 21 days apart, containing 10 µg of protein + 50 µg of Poly (I:C). Twenty-one days after the last dose, the humoral and cellular responses induced by vaccination were analyzed. In bronchoalveolar lavage (BALF), high levels of total anti-RBD (Figure 11, A) and anti-N (Figure 11, B) IgG antibodies were observed in mice with the respective protein and with the combination of N + RBD administered. The same was found in the serum of these animals, in which high rates of total anti-RBD IgG were detected in the groups that received RBD or N + RBD (Figure 11, C), whereas animals immunized with N or N + RBD showed exuberant levels of anti-N IgG antibodies (Figure 11, D).

To evaluate the cellular response, the splenocytes from mice were isolated and placed in culture with RBD or N antigens. After 48 hours, IFN-g was measured in the supernatant by using the enzyme-linked immunosorbent assay (ELISA). As demonstrated in Figure 11, E, mice immunized with RBD + Poly (I:C) were able to produce IFN-g after stimulation with RBD. Interestingly, splenocytes from animals immunized with N + RBD + Poly (I:C) produced even higher levels of IFN-g, when stimulated with *in vitro* RBD. On the other hand, when stimulated with the N protein, a higher rate of IFN-g was detected in mice with N + Poly (I:C) administered, when compared to N + RBD + Poly (I:C).

Next, the SpiN chimeric protein was tested. After expression and purification, the SpiN chimera was immunized in mice associated with Poly (I:C) adjuvant, following the same previously described protocol. Thirty after the last vaccine dose, IgG antibodies were measured in the serum. High levels of anti-N, anti-RBD and inactivated anti-SARS-CoV-2 total IgG antibodies were observed in mice immunized with SpiN + Poly (I:C) (Figures 12, A, B, C). Furthermore, a significant production of anti-N IgG1 and IgG2c subclasses (Figures 12, B and C, respectively), anti-RBD (Figures E and F) and inactivated anti-SARS-CoV-2 (Figures H and I) was detected. Interestingly, there was a predominance of anti-N IgG2c and RBD antibodies in relation to IgG1, suggesting a polarization of the Th1 response.

The cellular response induced by vaccination was also evaluated 30 days after the last vaccine dose, by measuring IFN-g and IL-10 in the splenocyte culture supernatant. It was observed that stimulation with RBD and N antigens induced the production of IFN-g by cells from mice immunized with SpiN + Poly (I:C) (Figure 13, A). Both stimuli also induced the production of IL-10, but at lower levels when compared to IFN-g (Figure 13, B).

In order to evaluate whether immunization with SpiN + Poly (I:C) is capable of providing protection against SARS-CoV-2 infection, hACE2 mice were immunized and challenged thirty days after the last vaccine dose. Figure 14, A shows that, unlike unvaccinated animals that received only Poly (I:C), the vaccinated animals did not lose weight after challenge with SARS-CoV-2. While 100% of vaccinated animals survived, all unvaccinated animals died by day 11 of infection with SARS-CoV-2 (Figure 14, B). Furthermore, the vaccinated animals showed undetectable viral load in the lung and brain, 11 days after the challenge with SARS-CoV-2 (Figure 14, C).

Figure 15 shows representative results of lung injury from vaccinated and unvaccinated animals challenged with SARS-CoV-2. The upper quadrants illustrate the lung tissue from an animal that received only the Poly (I:C) adjuvant. The lower quadrants illustrate the lung tissue from an animal vaccinated with SpiN + Poly (I:C). Hematoxylin and eosin staining (left quadrants) shows an intense inflammatory infiltrate in the unvaccinated animals, which is not observed in the vaccinated animals. Masson's Trichrome staining illustrates the formation of thrombi in the lung only of the unvaccinated animal challenged with SARS-CoV-2.

There can be concluded that the SpiN + Poly (I:C) vaccine formulation induces a robust humoral and cellular immune response that controls the viral load and significantly protects the inflammatory process and lung injury induced by infection with SARS-CoV-2 .

### EXAMPLE 10 - Humoral and cellular response of mice immunized with a vaccine formulation containing SpiN and squalene-based oil-in-water nanoemulsion

Subsequently, new immunogenicity tests were carried out, this time using a commercial squalene nanoemulsion called MF59 as an immunological adjuvant, which consists of an O/W nanoemulsion that comprises squalene as a substance with adjuvant activity. The selection of the adjuvant was based on the ability to induce effective immunity to influenza, which also infects humans through the respiratory tract. The squalene-based oil-in-water nanoemulsion lacks agonists for innate sensors, but is used in a commercially available vaccine with high immunogenic performance.

Mice were immunized with 10 µg of SpiN protein + 50 µL of squalene-based oil-in-water nanoemulsion. The data in Figure 16 demonstrate the ability of the SpiN protein, associated with the tested adjuvant, to induce a robust anti-N and anti-RBD antibody response. The cellular response was also evaluated in mice vaccinated with SpiN + MF59 squalene-based oil-in-water nanoemulsion. While the response to stimulation with the RBD protein (Figure 16b) was greater than the response to stimulation with the N protein (Figure 16c), both sets of protein-stimulated splenocytes produce high levels of IFN-γ (Figure 16d) and IL -10 when stimulated with SARS-CoV-2 proteins (Figure 16e).

In hamsters, using the same vaccination protocol, high levels of total IgG, IgG1 and IgG2c anti-N and anti-RBD antibodies were also observed (Figure 17 a-c).

Figures 16 (a-c) and 17 show that the association of SpiN with MF59 induces high levels of anti-N and anti-RBD total IgG in immunized mice and hamsters, respectively.

### EXAMPLE 11 - Protective effect of immunization with SpiN + MF59 in a murine COVID-19 model

Immunization with SpiN associated with squalene-based oil-in-water nanoemulsion in mice was evaluated by challenge against SARS-CoV-2, measured by viral load, evolution of body weight and histopathological analyses.

The protective effect of immunization with SpiN associated with MF59 (Figure 18) was evaluated in hACE-2 mice. Immunization with the SpiN protein associated with the squalene-based oil-in-water nanoemulsion protected 100% of the animals from clinical signs of the disease, such as raised hair, low motility and coma, as well as against weight loss and mortality (Figures 18a and 18b).

After a period of 11 days after the challenge in the vaccinated mice, a decrease in SARS-CoV-2 viral RNA was detected, contrasting with extremely high levels (10E4 PFU / 75 ng total / RNA) of viral load in the lungs (Figure 18c) and brains (Figure 18d) of non-immunized mice after 7-8 days of infection.

The histopathological examination showed results congruent with the clinical results. In the challenged placebo groups (upper panels), inoculated only with the squalene-based oil-in-water nanoemulsion, adjuvant, on day 5 post-infection, the lungs showed diffuse interstitial pneumonia characterized by a mixed inflammatory infiltrate of mononuclear and polymorphonuclear cells, accompanied by intense congestion, intra-alveolar exudate, hemorrhagic foci and areas of alveolar collapse (Figure 19). In vaccinated and challenged animals (lower panels), the preserved lung architecture is observed with the presence of peribronchoalveolar mononuclear infiltrate.

The vaccinated mice produced extremely high levels of anti-N and anti-RBD antibodies, and their role in mediating resistance to SARS-CoV-2 should not be ruled out. Thus, anti-N antibodies may act by mediating antibody-dependent cellular cytotoxicity (ADCC) or by promoting the internalization of host cells opsonized by macrophages. It is important to mention that SARS-CoV-2 does not replicate well in macrophages and there is no evidence that anti-N antibodies promote *in vitro* antibody-dependent enhancement (ADE) or *in vivo* replication of SARS-CoV-2 in tissues from vaccinated mice.

Furthermore, high levels of circulating neutralizing antibodies were observed in vaccinated animals after the challenge, which did not occur in unvaccinated animals (Figure 20).

Our results are consistent with the hypothesis that T lymphocytes are an important component of resistance to SARS-CoV-2 induced by vaccine formulations containing SpiN. Both the N protein and the RBD protein induced high levels of specific antibodies, and also promoted a T cell response and resistance to SARS-CoV-2. Furthermore, high levels of neutralizing antibodies were detected in animals vaccinated and infected with SARS-CoV-2.

In conclusion, immunization with the SpiN chimeric protein associated with both the squalene-based oil-in-water nanoemulsion (adjuvant) and Poly (I:C) as an adjuvant induces robust cellular and humoral immunity and protection against SARS-CoV-2. This protective immunity is believed to be largely mediated by T cells. Since T cell responses do not depend on a single epitope, non-silent point mutations are unlikely to severely impair the resistance induced by vaccination with the SpiN chimeric protein. Therefore, the N protein and the RBD region of the S protein, and more broadly the use of multiple T cell epitopes, should be considered in developing vaccines that overcome the genetic plasticity of SARS-CoV-2.

### EXAMPLE 12 - Evaluation of the immunogenicity of SpiN + MF59 in Callithrix penicillata primates

Ten adult *Callithrix penicillata* animals made up the study sample, six females and four males all healthy. The animals were provided by the Wild Animal Screening Center of the Brazilian Institute of the Environment and Renewable Natural Resources (IBAMA) to the Scientific Breeding Center for Research Purposes at the Federal University of Minas Gerais to carry out the study (CEUA/UFMG docket 54/2021). The animals underwent clinical evaluation before the experiment by a veterinarian who did not find any active clinical disease. Follow-up was initiated 6 days before the first sample collection and start of the immunization protocol (day -6), to establish a 6-day baseline and evaluate adverse effects of the vaccine. A 1.5 mL blood sample was taken from the right femoral vein on days 0 and 60.

Each animal received 0.3 mL of the immunizer, containing 10 µg of SpiN diluted 1:1 with MF59, intramuscularly (IM) in the right vastus medialis muscle on day 0 and day 30. They underwent temperature measurement at the abdominal region with an infrared thermometer and weighing throughout the experiment. During all interventions, they were also subjected to clinical evaluation, and the presence of skin reactions, pain, increased volume at the injection site and adjacent tissues, prostration, and lack of appetite were observed.

No systemic adverse effects were detected in *C. penicillata,* as suggested by the comparative evaluation of body weight loss and temperature variation. Animals immunized with SpiN and MF59 did not show variation in body temperature or significant weight loss (Figure 21). No prostration or lack of appetite was detected during the experiment, or any alterations or adverse reactions at the application site.

The results presented in Figure 22 show anti-N and anti-RBD antibody responses in these non-human primates vaccinated with SpiN + squalene-based oil-in-water nanoemulsion and SpiN + Poly (I.C.) (Figure 22).

### EXAMPLE 13 - Potency assay of the SpiN protein produced in different bacterial strains (pRARE and SHuffle)

For the production of polyclonal antibodies, rabbits were immunized with the N and RBD proteins according to the protocol described in LEENAARS and HENDRIKSEN (Critical steps in the production of polyclonal and monoclonal antibodies: evaluation and recommendations. ILAR journal, v. 46 , no. 3, p. 269-279, 2005).

The SpiN antigen, produced in two different bacterial strains (pRARE, two batches and SHuffle) was used to sensitize polystyrene plates (Costar, Ref. 2592), overnight at 4 °C with 100 pL/well of the antigen solution diluted (titration 6.5 to 800 ng/well) in carbonate buffer (pH 9.6). The solution was discarded and the wells were blocked for 2 h at 25 °C, using 250 µL of blocking solution (PBS1X + 1% BSA). After discarding the solution, 100 µl of polyclonal antibodies produced in rabbits (anti-N and anti-S/RBD, concentration 3 µg/mL and 22 µg/mL, respectively) diluted in PBS-T + 1% BSA were added and incubated at 37 °C (30 min). After five washes, 100 µl of secondary antibody (rabbit anti-IgG, Sigma, Ref: A0545) conjugated to the peroxidase enzyme diluted in sample diluent (1:50,000) was added and incubated at 37 °C (30 min). The plates were washed (1X PBS + 0.05% Tween 20) five times and incubated with 100 µl of TMB (3,3',5,5'-tetramethylbenzidine) (Science, Ref. One step) per well, sheltered from light for 15 minutes. The reaction was stopped with 100 µL of stop solution (0.5 M sulfuric acid), the absorbance measured on a Multiskan GO spectrophotometer (Thermo Fisher Scientific) at an optical density (O.D.) of 450 nm.

In Figure 23, it is possible to verify a high recognition capacity of the SpiN protein by anti-N (A) and anti-S/RBD (B) antibodies produced in rabbits. This capacity was even greater for protein produced in SHuffle, when compared to two different batches of the same protein produced in the pRARE strain.

### EXAMPLE 14 - Preparation of the immunogenic composition containing the protein defined by SEQ ID No. 1 and the squalene adjuvant

The nanoemulsion with adjuvant properties was prepared through an initial homogenization of the aqueous phase, composed of sodium citrate and citric acid (10 mM citrate buffer, pH 6.5) and polysorbate 80 with the oily phase, containing squalene and trioleate of sorbitan 85. After obtaining a conventional emulsion, the emulsion was homogenized under high pressure (using pressure between 800 and 1500 bar (80 and 150 MPa), 3 to 10 passing cycles) to obtain the nanoemulsion. Next, the adjuvant and the buffer solution were subjected to sterilizing filtration through a 0.22 µm sterilizing filter and mixed with the sterile SpiN protein solution (SEQ ID No. 1) in pre-established proportions, in order to obtain the final SpiN protein concentrations between 10 µg/mL and 200 µg/mL in the immunogenic composition. The formulation was filled into glass flasks in a classified area. In the examples that follow, this composition will be called SpiN-squalene nanoemulsion.

### EXAMPLE 15 - Evaluation of particle size, zeta potential and polydispersity index of the immunogenic composition

The comparability evaluation between the products SpiN-squalene nanoemulsion and SpiN-AddaVax was carried out by testing particle size, polydispersity index and zeta potential, using the Zetasizer Nano ZS-90 equipment, Malvern. The sample was diluted 100 times in purified water by performing 3 independent replicates of each batch of SpiN-squalene nanoemulsion (21/1880 and 21/1879) and SpiN-AddaVax (CT 181021/5805-42-03; CT 181021/5805-43-01; CT 181021/5805-43-02). The transmission electron cryo-microscopy (cryo-TEM) was used to study the morphology of the nanoemulsion in the presence of the SpiN protein. The analyses were carried out on a Tecnai G2-12 - FEI SpiritBiotwin 120 kV microscope, at the UFMG Microscopy Center. The samples used in this analysis were SpiN-squalene nanoemulsion 200 ug/mL batch 21/1879 and SpiN-AddaVax 200 ug/mL batch (CT 181021/5805-42-03), prepared by the immersion freezing technique, spreading the sample into a thin film through a grid and, then, quickly submerging the same in liquid ethane. The determination of the average diameter was carried out by analyzing 100 nanoparticles, using the Image J software. For statistical analysis to evaluate the values of size, polydispersity index and zeta potential, the one-way analysis of variance (ANOVA) test was applied followed by the Tukey test. For microscopy data, the statistical analyses were performed using the Student's t-test. The analyses of normality and homogeneity of variance were performed using the D'Agostino and Pearson and Bartlett tests, respectively. Differences were considered significant when the p value was less than 0.05. When evaluating the comparison results between SpiN-squalene nanoemulsion and SpiN-AddaVax (Table 2), it is observed that there is no statistical difference between them in relation to particle size and polydispersity index, suggesting similarity between the formulations. However, the zeta potential of SpiN-AddaVax 200 µg/mL was statistically different from SpiN-squalene nanoemulsion 200 µg/mL, as well as both formulations at the concentration of 100 ug/mL (SpiN-AddaVax and SpiN-nanoemulsion of squalene), with a value close to -30 mV. However, all formulations presented zeta potential within the adopted acceptance limit and are capable of providing high stability to the system.

**Table 2 - Particle size, polydispersity index and zeta potential of SpiN-squalene nanoemulsion and SpiN-AddaVax.**

| | **SpiN-squalene nanoemulsion 100 µg/mL** | **SpiN-AddaVax 100 µg/mL** | **SpiN-squalene nanoemulsion 200 µg/mL** | **SpiN-AddaVax 200 µg/mL** |
|---|---|---|---|---|
| Size | 169.5 ± 2.35 | 154 ± 5.18 | 183.3 ± 5.83 | 195.4 ± 16.01 |
| PI | 0.09 ± 0.01 | 0.06 ± 0.02 | 0.07 ± 0.02 | 0.07 ± 0.02 |
| Zeta potential | -38.7 ± 0.76 | -38.9 ± 2.78 | -38.4 ± 0.86 | -29.7 ± 1.73* |

| | | | | |
|---|---|---|---|---|
| Data expressed as mean ± standard deviation (n = 3 batches). p < 0.05 (Tukey test). *Difference in relation to SpiN-AddaVax 200 µg/mL. #Difference in relation to SpiN-AddaVax 100 µg/mL. | | | | |

The SpiN-squalene nanoemulsion and SpiN-AddaVax samples were in the form of round globules, with a smooth, uniform surface and with an average size below 200 nm (Figure 24), as observed for the adjuvant in absence of protein. When analyzing the size distribution graph (Figure 25), it is possible to observe that there was no statistical difference between SpiN-squalene nanoemulsion and SpiN-AddaVax. Additionally, it is noted that for SpiN-squalene nanoemulsion there is a greater proportion of smaller particles. This data is consistent with dynamic light scattering (DLS) data, where a smaller average size is also observed for the SpiN-squalene nanoemulsion. The slightly larger average size observed for the DLS technique in relation to cryomicroscopy can be explained by the difference in techniques. While the DLS technique provides the result from the measurement of the hydrodynamic radius, in transmission electron microscopy the dimensions are measured directly from the XY distances in the images obtained, providing the 'true radius' of the particle. After the studies conducted, there can be concluded that the SpiN-squalene nanoemulsion formulation was similar to the SpiN-AddaVax mixture in terms of size, polydispersity index and globule morphology, with no statistical difference between them. Additionally, all formulations presented zeta potential with average values of -30 to -38 mV, values capable of providing electronic stabilization to the nanoemulsion globules. Therefore, this evaluation, together with the immunogenicity data, allows the obtention of evidence to prove the equivalence of the two formulations.

### EXAMPLE 16 - Physicochemical and microbiological characterization of the immunogenic composition

The formulation has the appearance of an opalescent and homogeneous white emulsion after gentle stirring; pH between 7.0 and 8.0; average particle diameter between 150 and 245 nm, polydispersity index less than 0.3, zeta potential less than 0.30 mV, viscosity between 1.0 cP and 1.8 cP, osmolality between 900 and 1500 mOsm/kg, and squalene content between 885 and 110% of the amount declared on the label. It presents specific biological activity of 1.5 x 10³ to 9.50 x 10³ IU/mg for the anti-N antibody and the anti-RBD antibody; *in vivo* potency result showing total IgG antibody titers greater than or equal to 10² after the first dose and greater than or equal to 10³ after the second dose. The absence of microorganisms was observed in the sterility test; endotoxin values below 20 EU/mL, absence of visible particle contamination; subvisible particles ≥ 10 um with a maximum of 3000 particles / vial; and particles greater than or equal to 25 um with a maximum of 300 particles/vial. It was found that the immunogenic composition presents satisfactory results when stored at a controlled temperature of 2 °C to 8 °C for at least 9 months.

**Table 3 - Specifications of tests, methods and acceptance criteria used as a reference to evaluate the quality of the SpiN-squalene nanoemulsion composition during the long-term stability study and the respective results obtained throughout the study.**

| **Specifications** | | **Test Interval** | | | |
|---|---|---|---|---|---|
| **Test** | **Acceptance Criteria** | **Initial Time 11/17/21** | **3 Months 02/10/22** | **6 Months 05/16/22** | **9 Months 08/14/22** |
| Appearance | Opalescent and homogeneous white emulsion after gentle stirring | Meets the test | Meets the test | Meets the test | Meets the test |
| pH | Between 7.0 and 8.0 | 7.94 ± 0.02 | 7.52 ± 0.03 | 7.53 ± 0.03 | 7.44 ± 0.04 |
| Particle Size (nm) | Between 165.0 and 245.0 d.nm | 190.0 | 193.3 | 186.6 | 191.2 |
| Polydispersity index | Less than 0.3 | 0.068 | 0.115 | 0.068 | 0.092 |
| Zeta potential (mV) | Less than -30 mV | -37.5 | -35.2 | -34.5 | -41.4 |
| Viscosity (Cp) | Between 1.00 and 1.80 Cp (246 rpm, ambient temperature) | 1.31 | Not applicable | 1.58 | 1.35 |
| Osmolality (mosmol/kg) | Between 900 and 1500 mOsm/kg | 992 ± 57 | Not applicable | 1034 ± 15 | 1023 ± 17 |
| Squalene dosage | Contains a minimum of 85.0% and a maximum of 110.0% of the amount of squalene | 99.99 ± 0.68 | 101.78 ± 2.19 | 102.10 ± 2.61 | 98.43 ± 0.07 |
| Biological activity of SpiN by ELISA | 1.5 x 10³ to 9.5 x 10³ IU/mg for the anti-N antibody and for the anti-RBD antibody | Anti-N: 3.6 x 10³ | Anti-N: 3.2 x 10³ | Anti-N: 2.6 x 10³ | Anti-N: 4.5 x 10³ |
| | | Anti-RBD: 2.6 x 10³ | Anti-RBD: 2.5 x 10³ | Anti-RBD: 2.2 x 10³ | Anti-RBD: 2.2 x 10³ |
| Purity by DSS-EGPA | The electrophoretogram obtained with the problem solution does not present any other band, besides the main band of SpiN equivalent to 68 kDa, that is more intense than the main band obtained with the | - | - | Meets the test | Meets the test |
| | standard solution diluted to 20%. | | | | |
| Sterility | Absence of viable microorganisms | Meets the test | Not applicable | Not applicable | ** |
| Bacterial endotoxins | Maximum 20 EU/mL | < 5.0 EU/mL | Not applicable | Not applicable | ** |
| Determination of potency | Total IgG antibody titers greater than or equal to 10², after the first dose and greater than or equal to 10³, after the second dose. | Anti-N: 10⁵ | Not applicable | Anti-N: 10⁵ | *1^{st} dose*: |
| | | | | | Anti N: 10⁵ |
| | | Anti-SpiN: 10⁵ | | Anti-SpiN: 10⁵ | Anti SpiN: 10³ |
| | | Anti-RBD: 10⁴ | | Anti-RBD: 10³ | Anti RBD: 10² |
| | | | | | *2^{nd} dose*: |
| | | | | | In progress |
| Subvisible particle contamination | Particles ≥ 10 µm: maximum 3000 particles per vial. | Particles 2 10 µm: 02 | Not applicable | Not applicable | ** |
| | Particles ≥ 25 µm: maximum 300 particles per vial. | Particles ≥ 25 µm: 03 | | | |
| Contamination by visible particles | Absence of visible particles | Complies with | Not applicable | Not applicable | ** |

Squalene measurement by gas chromatography was carried out on days 0, 90, 180 and 270. The values remained within the adopted specification limit, with no significant difference being observed between the same (Figure 26). The squalene quantification analysis by gas chromatography allowed obtaining the sample retention time (4.5 min), which corresponds to the retention time of the standard, allowing the confirmation of the identity of the squalene present in the formulation of the immunogenic composition.

The evaluation of purity, including possible degradation or cleavage products of SpiN in the immunogenic composition, stored at 5 °C ± 3 °C, was evaluated by polyacrylamide gel electrophoresis under denaturing conditions using sodium dodecyl sulfate (DSS-EGPA), with a standardized application of 2 µg of SpiN on the gel, at 180 and 270 days. The result presented in Figure 26 demonstrates the presence of a similar band profile in samples stored for 180 and 270 days, that is, a band with migration close to the 68 kDa region, which corresponds to the expected molecular mass for the SpiN protein, indicating stability of IFA in the immunogenic composition. Furthermore, the presence of additional bands was not observed in the gel, indicating the absence of cleavage products, even after 270 days of storage (Figure 27) and proving the stability profile at the end of the 9-month period.

### EXAMPLE 17 - Evaluation of the in vitro activity of the immunogenic composition

The ELISA results obtained with the immunogenic composition stored at different times were compared. For this assay to evaluate and prove identity and activity, a serial dilution of the composition and SpiN protein stored at -20 °C (positive control) was carried out, starting at 800 ng with an experimental design of eight (8) dilutions (8 µg/mL to 0.625 pg/mL). After applying the protein or composition to the plate wells, polyclonal primary antibodies (anti-RED and anti-N), generated in rabbits against the respective recombinant proteins, were added to the plate to recognize the N and RBD portions of the SpiN protein. As shown in Figure 28, these antibodies recognized the two portions of SpiN (N and RBD), contained in the SpiN-squalene nanoemulsion formulation stored at 5 °C ± 3 °C, for 270 days, with intensities similar to those obtained with the immunogenic composition stored at 5 °C ± 3 °C for 90 and 180 days. The average values of the specific anti-RBD and anti-N biological activity remain within the range of the established acceptance criteria, attesting to the maintenance and stability of the biological activity of the composition stored for up to 270 days. It is worth mentioning that the assay allows the identity of the IFA to be evaluated and is correlated to the concentration of the SpiN protein in the formulation. The maintenance of the biological activity of the adjuvanted composition (Figure 29), after 270 days of storage, at 5 °C ± 3 °C, was corroborated by the results obtained in the potency test, whose determined anti-N, anti-RBD and anti-SpiN antibody titers presented values within the adopted acceptance range.

### EXAMPLE 18 - Evaluation of the in vivo activity of the immunogenic composition

After storing the composition for 180 days at 5 °C ± 3 °C, two doses of 20 µg of the composition were injected into mice, 21 days apart, and antibody levels were evaluated 30 days after the second dose (Figure 30). It is observed that there was no statistical difference between the potency of the product, when comparing the values obtained at the initial time (D0) and after storage for 180 days at 5 °C ± 3 °C (D180), considering the anti-N and anti-RED titers. For the anti-SpiN ELISA, there was a small drop in antibody titers, but the potency remained in the order of 10⁵. Thus, the product was considered stable and immunogenic for at least 180 days, since the average of the potencies, evaluated by the levels of total IgG against the three analyzed antigens, was equal to or greater than 103, which corresponds to the pre-determined acceptance criterion, after two doses of the product, for the storage period. The potency test of the composition stored for 270 days was carried out after the first dose. For this, a dose of 20 µg of the composition stored for 270 days at 5 °C ± 3 °C was injected into mice and the antibody levels were evaluated after 21 days. As shown in Figure 30, there was no statistical difference between the potency of the product at the initial time (D0) and after storage for 270 days at 5 °C ± 3 °C (D270), considering the anti-N and anti-SpiN titers. For the anti-RBD ELISA, there was a drop in antibody titers, but even so, the potency remained in the order of 10². Since the acceptance criterion for this test is equal to or greater than 10², after administration of the first dose, the product was considered stable and immunogenic for 9 months.

## Claims

1. **A CHIMERICAL PROTEIN PRODUCTION PROCESS, characterized in that** it comprises the following steps:
a. Cloning the DNA, defined by SEQ ID No. 2, encoding the protein defined by SEQ ID No. 1, into an expression vector;
b. Transforming *E. coli* bacteria with the construction obtained in "a" and cultivating in an appropriate medium, at a temperature of 36.5 to 37.5 °C, under stirring at 180 to 200 rpm, for 3 to 4 hours;
c. Inducing the expression of the protein defined by SEQ ID No. 1 by adding IPTG to the medium, and maintaining cultivation under stirring for a period of 15 h to 24 h, at 36.5 to 37.5 °C;
d. Lysing the cell precipitate from the cultivation obtained in "c", using a lysis buffer, which comprises a buffering agent with a pH between 7 and 8, a cocktail of protease inhibitors and reducing agents, by passing the sample through homogenizing equipment by pressure maintaining 15,000 to 20,000 psi (103.42 to 137.90 MPa) for 5 to 20 min;
e. Submitting the lysate obtained in "d" to centrifugation at 35,000 g to 45,000 g for 20 to 40 min;
f. Performing 2 washing steps of the insoluble fraction obtained in "e" using a buffer containing 0.5 to 1 M urea, pH 7 to 8, through resuspension and centrifugation at 35,000 g to 45,000 g for 20 to 40 min;
g. Performing the precipitate resuspension step containing the washed insoluble fraction obtained in "f" in a buffer containing 6 to 8 M urea and protease inhibitor cocktail by stirring at 4 to 6 °C for approximately 18 to 24 h;
h. Submitting the resuspension obtained in "g" to centrifugation at 35,000 g to 45,000 g for 20 to 40 min;
i. Submitting the solubilized sample obtained in "h" to two steps of ion exchange chromatography, the first in an anionic column, and then the material not bound in the anionic column is applied to a cationic column, at pH 7 to 8, in the presence of 6 to 8 M urea;
j. Submitting the fractions with the highest concentration of the SpiN protein obtained in "i" to a desalting chromatography, to the remotion of the urea and substitution for a buffer containing 50 to 150 mM urea, pH 10.5 to 11.5.

2. **THE PROCESS according to claim 1, characterized in that, in step "a",** the expression vector is the pET24a prokaryotic vector.

3. **THE PROCESS according to claim 1, characterized in that, in step "b",** the appropriate medium is such as LB medium with 25 to 100 ug/mL of kanamycin.

4. **THE PROCESS according to claim 1, characterized in that, in step "c",** the IPTG is added to a final concentration of 0.1 to 1 mM, when the optical density at 600 nm reaches a value of 0.3 to 0.6.

5. **THE PROCESS according to claim 1, characterized in that, in step "f",** the washing is carried out with 80 to 100 mM Tris buffer, 0.5 to 1 M urea, 5 to 10 mM EDTA, 5 to 10% glycerol, 1 to 2 triton x-100.

6. **THE PROCESS according to claim 1, characterized in that, in step "g",** the washing is carried out with 25 to 100 mM phosphate buffer, 5 to 10% glycerol, 6 to 8 M urea, 0.05 to 0.1% triton x-100, 1 to 10 mM DTT, 1 to 10 mM benzamidine, 1 to 5 mM PMSF and protease inhibitor cocktail.

7. **THE PROCESS according to claim 1, characterized in that, in step "j",** the replacement buffer is 25 to 100 mM phosphate buffer, 5 to 10% glycerol, 50 to 150 mM urea, 15 to 35 mM NaOH, 100 to 200 mM NaCl, pH 10.5 to 11.5.

8. **A CHIMERICAL PROTEIN obtained by the process defined in claim 1, characterized in that** it consists of the amino acid sequence defined by SEQ ID No. 1.

9. **A GENE FOR THE CHIMERIC PROTEIN defined in claim 2, characterized in that** it consists of the nucleotide sequence defined by SEQ ID No. 2.

10. **AN IMMUNOGENIC COMPOSITION AGAINST SARS-CoV2, characterized in that** it comprises a chimeric protein defined by SEQ ID No. 1 and adjuvants.

11. **THE IMMUNOGENIC COMPOSITION according to claim 10, characterized in that** the adjuvants are selected from the group comprising polyinosinic:polycytidylic acid and derivatives, water and oil emulsion or alum with unmethylated CpG oligonucleotide, water and oil emulsion or alum with lipid A, squalene and derivatives, synthetic TLR4 agonist, liposomes associated with TLR unmethylated CpG or TLR4 agonist, Montanide and Monophosphoryl Lipid A, or another adjuvant that induces a Th1-type immune response.

12. **THE IMMUNOGENIC COMPOSITION according to claim 10, characterized in that** it comprises 1 µg to 1 mg of the protein defined by SEQ ID No. 1 and 1 µg to 10 mg of the polyinosinic:polycytidylic acid adjuvant, or 1 µg to 10 mg of CpG and 10% to 90% m/v of Alum, per dose.

13. **THE IMMUNOGENIC COMPOSITION according to claim 10, characterized in that** it comprises 10 µg to 200 µg of the protein defined by SEQ ID No. 1 per dose, and 5 µg to 5 mg of the polyinosinic:polycytidylic acid adjuvant per dose, or 18 µg of CpG and 30% m/v of Alum, per dose.

14. **THE IMMUNOGENIC COMPOSITION according to claim 10, characterized in that** it comprises 10 µg to 500 µg of the protein defined by SEQ ID No. 1, and 1 mg to 20 mg of the squalene adjuvant, per dose.

15. **A USE OF THE CHIMERIC PROTEIN defined in claim 8, characterized in that** it is for the production of a vaccine for the prophylaxis and prevention of infection with and moderate and severe forms of COVID-19.

16. **A USE OF THE IMMUNOGENIC COMPOSITION defined in claim 10, characterized in that** it is for the production of a vaccine for the prophylaxis and prevention of infection with and moderate and severe forms of COVID-19.
